# EUROPÄISCHE PATENTSCHRIFT

(11) **EP 0 660 711 B1**
(45) Veröffentlichungstag und Bekanntmachung des Hinweises auf die Patenterteilung: **31.03.1999**
(21) Anmeldenummer: 93920652.0
(22) Anmeldetag: 14.09.1993
(51) Int. Cl.: A61K 31/38, A61K 31/40, A61K 31/415, A61K 31/44, A61K 31/50, A61K 31/505, A61K 31/52, A61K 31/54

(54) **NEUE VERWENDUNG VON INHIBITOREN DER PHOSPHODIESTERASE IV**
NOVEL USE OF INHIBITORS OF PHOSPHODIESTERSASE IV
NOUVELLE UTILISATION D'INHIBITEURS DE LA PHOSPHODIESTERASE IV

(30) Priorität: 14.09.1992 DE 4230755; 17.07.1993 DE 4324571
(43) Veröffentlichungstag der Anmeldung: 05.07.1995
(73) Patentinhaber: FORSSMANN, Wolf-Georg, Prof. Dr., 30625 Hannover (DE)
(72) Erfinder: STIEF, Christian, D-30966 Hemmingen 1 (DE); STROHMEYER, Torsten, D-12161 Berlin (DE); FORSSMANN, Wolf-Georg, D-30175 Hannover (DE); MEYER, Markus, D-30655 Hannover (DE); SCHULZ-KNAPPE, Peter, D-30519 Hannover (DE); TAHER, Akmal, Djakarta 12710 (ID)
(74) Vertreter: Meyers, Hans-Wilhelm, Dr.Dipl.-Chem.
(86) Internationale Anmeldenummer: DE9300892
(87) Internationale Veröffentlichungsnummer: WO9406423

(56) Entgegenhaltungen:
- TRENDS IN PHARMACOLOGICAL SCIENCE Bd. 12, Nr. 1 , Januar 1991 Seiten 19 - 27 NICHOLSON, C.D. ET AL 'DIFFERENTIAL MODULATION OF TISSUE FUNCTION AND THERAPEUTIC POTENTIAL OF SELECTIVE INHIBITORS OF CYCLIC NUCLEOTIDE PHOSPHODIESTERASE ISOENZYMES' in der Anmeldung erwähnt
- THE JOURNAL OF PHARMACOLOGY AND EXPERIMENTAL THERAPEUTICS Bd. 247, Nr. 2 , November 1988 Seiten 630 - 634 WEISS, R.M. ET AL 'INSULIN ACTIVATION OF CYCLIC AMP PHOSPHODIESTERASE IN INTACT URETERAL SEGMENTS' in der Anmeldung erwähnt
- THE TOHOKU JOURNAL OF EXPERIMENTAL MEDICINE Bd. 151, Nr. 2 , Februar 1987 Seiten 201 - 204 MORITA, T. ET AL 'THE TIME COURSE OF CHANGES IN FORCE AND CYCLIC AMP LEVELS PRODUCED BY ISOPROTERENOL AND FORSKOLIN IN ISOLATED RABBIT DETRUSOR MUSCLE'

## Beschreibung

Die Erfindung betrifft die Verwendung von Inhibitoren der Phosphodiesterase IV (sPDE IV) zur Herstellung von Arzneimitteln zur Modulation der Motilität und Peristaltik der Hohlorgane des Urogenital- und Gastrointestinaltraktes sowie deren Verwendung als Hilfsstoffe für Kontrastmitteluntersuchungen des Urogenital- und Gastrointestinaltraktes.

Die physiologische Informationsübertragung zur Relaxation (Erschlaffung) glatter Muskelzellen wird durch Überträgerstoffe des Blutes (Hormone) oder der Nerven (Neurotransmitter) bewirkt. Diese Neurotransmitter bewirken innerhalb der glatten Muskelzelle einen Anstieg von cAMP und cGMP, was eine Relaxation zur Folge hat. cAMP und cGMP wiederum werden durch Phosphodiesterasen (PDE) abgebaut. Inhibitoren der PDE wiederum vermindern den Abbau von cAMP und cGMP, was zu einem Anstieg dieser Moleküle innerhalb der Zelle und dadurch zu einer Relaxation der glatten Muskelzelle führt. Dieses ist beispielsweise beschrieben von Torphy, Undem in Thorax 46, 512, 1991.

Aus dieser Veröffentlichung und aus TIPS 12, 19, 1991 sowie Br. J. Pharmacol. 104, 471, 1991 ist weiterhin eine Unterscheidung der PDE in verschiedene Unteresterasen, die spezifischen Phosphodiesterasen (sPDE), bekannt. Dabei werden fünf verschiedene sPDE unterschieden, die in den einzelnen Organen und Organsystemen unterschiedlich verteilt sind und je nach Verteilung in der Zelle eine verschieden starke Wirksamkeit besitzen. In den genannten Veröffentlichungen sowie in J. Histochem. Cytochem. 35, 72, 1987, J. Urol. 139, 1988 und J. Pharmacol. Exp. Therap. 247, 630, 1988 wird auch das Vorkommen der verschiedenen Isoenzyme in diversen Geweben diskutiert, unter anderem auch das Vorkommen von sPDE I im Ureter (Harnleiter).

Nieren- oder Harnleiterkoliken besitzen nach Altwein und Jacobi, Urologie, Enka Verlag Stuttgart, 1987, den Charakter einer Volkskrankheit. Der Kolikschmerz entsteht durch einen intrarenalen Druckanstieg durch den gestörten Urintransport sowie durch lokale Spasmen, die den spontanen Abgang eines Steines behindern. Es kommt zu einer Implaktierung des Steines, die die Gefahr der Stauung und der damit verbundenen schwerwiegenden Komplikationen bewirkt. Wird bei Steinpatienten kein spontanabgang eventuell mit Hilfe von Pharmaka erreicht, muß man sich zu einem invasiven Vorgehen entschließen.

Eine Behandlung dieser Erkrankungen erfolgt derzeit symptomatisch durch starkwirkende Analgetika zur Schmerzlinderung. Eine medikamentöse Therapie der Ursachen einer Kolik ist bisher nicht möglich, da keine die glatte Muskulatur relaxierenden Substanzen ohne begleitende unerwünschte, gravierende systemische Nebenwirkungen (Blutdruckabfall, Übelkeit) bekannt sind.

Aufgabe der Erfindung ist daher die Bereitstellung hochwirksamer spezifischer Therapeutika zur Modulation der Motilität und Peristaltik der Hohlorgane des Urogenital- und Gastrointestinaltraktes, die keine Nebenwirkungen verursachen. Überraschenderweise wurde nun gefunden, daß durch die Inhibition der sPDE IV die Modulation der Motilität und Peristaltik der Hohlorgane des Urogenital- und Gastrointestinaltraktes beeinflußt wird. Eine gezielte Hemmung dieses Isoenzyms wirkt auf die glatte Muskulatur relaxierend und ermöglicht die Behandlung von Erkrankungen des Urogenital- und Gastrointestinaltraktes, bei denen die Relaxation der glatten Muskulatur erwünscht ist, wie beispielsweise die Behandung von Nieren- und Uretererkrankungen, die Behandlung von Erkrankungen der Gallenwege oder Störungen des Magen-Darmtraktes die Störungen im Sinne des Colon irritabile oder Magenkrämpfe. Beispielsweise wirkt eine gezielte Hemmung dieses Isoenzyms auf Tonus und Peristaltik des partiell oder völlig okkludierten Harnleiters relaxierend. Durch die Relaxation des Ureters kann der Steinabgang gefördert und beschleunigt und eine Behandlung von Koliken ermöglicht werden. Auf Grund der relaxierenden Wirkung auf die glatte Muskulatur können Inhibitoren der sPDE IV in Kombination mit den üblichen Kontrastmitteln zur Verbesserung der Diagnose der vorstehend genannten Erkrankungen verwendet werden. Die Verabreichung der Inhibitoren der sPDE IV kann kurz vor, nach oder gleichzeitig mit einer Gabe von Röntgen-, Ultraschall- oder NMR-Kontrastmitteln erfolgen.

Gegenstand der Erfindung ist daher die Verwendung von spezifischen Inhibitoren der sPDE IV zur Herstellung von Arzneimitteln zur Modulation der Motilität und Peristaltik der Hohlorgane des Urogenital- und Gastrointestinaltraktes, sPDE IV-Inhibitoren enthaltende Arzneimittel für die genannte Aufgabe sowie deren Verwendung als Hilfsstoffe bei Diagnostika. Bevorzugte Inhibitoren der sPDEIV sind beispielsweise:
1.) 1,3-Dibutyl-3,7-dihydro-7-(2-oxopropyl)-1H-purin-2,6-dion (Denbufylline, BRL 30892),
2.) 4-[(3-Butoxy-4-methoxyphenyl)methyl]-2-imidazolidionon (Ro 20-1724),
3.) 4-[3-(Cyclopentyloxy)-4-methoxyphenyl]-2-pyrrolidinon (Rolipram, ZK 62711),
4.) 5,6-Diethoxybenzo[b]thiophen-2-carbonsäure (Tibenelast, LY 186655),
5.) 3-Ethyl-1-(3-nitrophenyl)-2,4(1H,3H)-chinazolinedion (Nitraquazone, TVX 2706),
6.) 6-(3,6-Dihydro-6-methyl-2-oxo-2H-1,3,4-thiadiazin-5-yl)-1-(3,4-dimethoxybenzoyl)-1,2,3,4-tetrahydro-4,4-dimethylchinolin (EMD 54622),
7.) 1-Ethyl-4-[(1-methylethyliden)hydrazino]-1H-pyrazolo[3,4-b]pyidin-5-carbonsäure ethylester (Etazolate),
8.) N-Hydroxy-5,6-dimethoxy-benzo[b]thiophen-2-carboximidamid (Org 30029),
9.) 2-Amino-6-methyl-4-propyl-(1,2,4)triazolo[1,5-a]pyrimidin-5(4H)-on (ICI 63197) oder
10.) 6-(4-(Difluoromethoxy)-3-methoxyphenyl]-3(2H)-pyridazinon (Zardaverine)
   sowie deren pharmakologisch verträglichen Salze.

Die Verbindungen sind bekannt als wirksam z.B. bei Erkrankungen der Atemwege, zur Entzündungshemmung oder bei Erkrankungen des Zentralnervensystems.

Als bevorzugter sPDE IV-Inhibitor ist razemisches oder optisch aktives Rolipram zu betrachten, dessen Herstellung nach US-Patent No. 4.193.626 oder nach WO 92/06077 erfolgen kann.

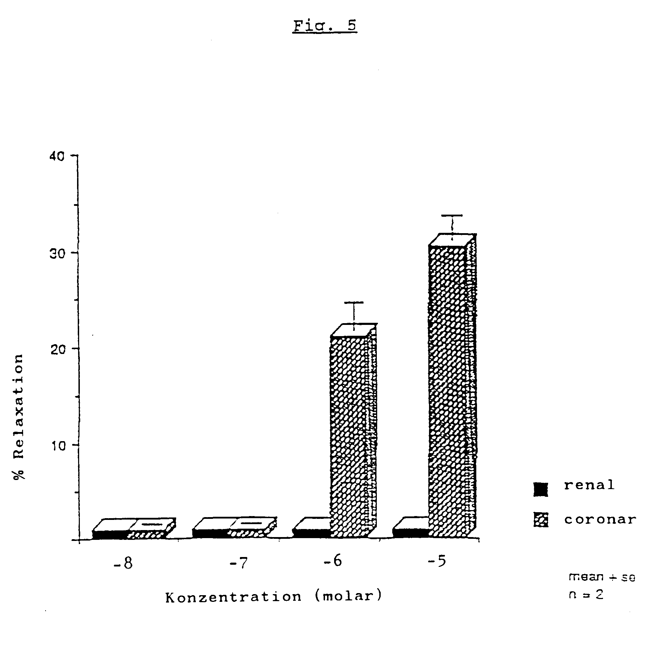

Bereits die Applikation geringster Dosierungen eines spezifischen Inhibitors z.B. des sPDE IV-Inhibitors Rolipram in einer Dosierung von 10⁻⁷ mol/l (Fig. 4) relaxiert den Ureter, ohne daß nennenswerte Effekte an anderen Organen, insbesondere an Gefäßen, zu beobachten sind. Dies wurde an humanen Ureter-Streifen in vitro gezeigt.

Zu den gleichen Ergebnissen führten Untersuchungen am lebenden Tier (Kaninchen). Auch hier wurde der Harnleiter durch intravenöse Gaben von sPDE IV-Inhibitoren wie Rolipram weitgestellt, ohne daß Nebenwirkungen wie Blutdruckabfall auftraten.

Im Gegensatz dazu führte die Gabe des unspezifischen PDE-Hemmers Papaverin zu ausgeprägten Kreislaufnebenwirkungen.

Unsere Untersuchungsergebnisse zeigen, daß spezifische Phosphodiesterase-IV-Inhibitoren und insbesondere razemisches oder optisch aktives Rolipram zur Modulation der Motilität und Peristaltik des Urogenital- und Gastrointestinaltraktes verwendet werden können. Durch die Relaxation der glatten Muskulatur wird beispielsweise der Abgang von Steinen erleichtert und Koliken werden verhindert. Beispielsweise können sie durch die Relaxation des Ureters zur Beschleunigung und Erleichterung des Abganges von Nieren- und Ureter-Steinen verwendet werden sowie Koliken verhindern oder beenden.

Zur Herstellung der Arzneimittel wird neben den üblichen Hilfsmitteln, Träger- und Zusatzstofzen eine wirksame Dosis der Inhibitoren der sPDE IV oder deren Salze in den oben genannten Indikationen verwendet.

Die pharmakologisch verträglichen Salze werden in üblicher Weise durch Neutralisation der Basen mit anorganischen oder organischen Säuren erhalten. Als anorganische Säuren kommen zum Beispiel Salzsäure, Schwefelsäure, Phosphorsäure oder Bromwasserstoffsäure, als organische Säuren zum Beispiel Carbon-, Sulfo- oder Sulfonsäuren wie Essigsäure, Weinsäure, Milchsäure, Propionsäure, Glykolsäure, Malonsäure, Maleinsäure, Fumarsäure, Gerbsäure, Succinsäure, Alginsäure, Benzoesäure, 2-Phenoxybenzoesäure, 2-Acetoxybenzoesäure, Zimtsäure, Mandelsäure, Zitronensäure, Äpfelsäure, Salicylsäure, 3-Aminosalicylsäure, Ascorbinsäure, Embonsäure, Nicotinsäure, Isonicotinsäure, Oxalsäure, Aminosäuren, Methansulfonsäure, Ethansulfonsäure, 2-Hydroxyethansulfonsäure, Ethan-1,2-disulfonsäure, Benzolsulfonsäure, 4-Methylbenzolsulfonsäure oder Naphthalin-2-sulfonsäure in Frage.

Die Dosierung der Wirkstoffe kann je nach Verabfolgungsweg, Alter, Gewicht des Patienten, Art und Schwere der zu behandelnden Erkrankung und ähnlichen Faktoren variieren.

Die tägliche Dosis kann als einmal zu verabreichende Einzeldosis oder unterteilt in 2 oder mehrere Tagesdosen gegeben werden und entspricht im allgemeinen der Dosis, die von der jeweiligen Verbindung als wirksame Dosis bekannt ist.

Die tägliche Gesamtdosis von Rolipram beträgt üblicherweise 0,001 - 10 mg/pro Person, vorzugsweise 0,01 - 5 mg/pro Person. Wird (-) Rolipram als Wirkstoff verabfolgt, so beträgt die tägliche Dosis vorzugsweise 0,001 - 5 mg. Durch mehrtägige Auftitration kann die Gesamtdosis jedoch bei Bedarf signifikant erhöht werden. Als Applikationsform kommen orale, intravenöse, intraluminäre Zubereitungen in Frage. Letztere sind vor allen Lösungen und Zubereitungen wie sie auch für die parenterale Applikation Anwendung finden.

Zubereitungen zur parenteralen Applikation können in separaten Dosiseinheitsformen wie z. B. Ampullen oder Vials vorliegen. Vorzugsweise werden Lösungen des Wirkstoffes verwendet, bevorzugt wässrige Lösungen und vor allem isotonische Lösungen, aber auch Suspensionen. Diese Injektionsformen können als Fertigpräparat zur Verfügung gestellt werden oder erst direkt vor der Anwendung durch Mischen der wirksamen Verbindung, zum Beispiel des Lyophilisats, gegebenenfalls mit weiteren festen Trägerstoffen, mit dem gewünschten Lösungs- oder Suspensionsmittel zubereitet werden.

Zur oralen Anwendung kommen die üblichen galenischen Zubereitungsformen wie Tabletten, Dragees, Kapseln, dispergierbare Pulver, Granulate, wässerige oder ölige Suspensionen, Sirupe, Säfte oder Tropfen.

Feste Arzneiformen können inerte Hilfs- und Trägerstoffe enthalten, wie z.B. Calciumcarbonat, Calciumphosphat, Natriumphosphat, Lactose, Stärke, Mannit, Alginate, Gelatine, Guar-gummi, Magnesium- oder Aluminiumstearat, Methylcellulose, Talkum, hochdisperse Kieselsäuren, Silikonöl, höhermolekulare Fettsäuren (wie Stearinsäure), Gelatine, Agar-Agar oder pflanzliche oder tierische Fette und Öle, feste hochmolekulare Polymere (wie Polyäthylenglykol); für orale Applikation geeignete Zubereitungen können gewünschtenfalls zusätzliche Geschmacks- und/oder Süßstoffe enthalten.

Flüssige Arzneiformen können sterilisiert sein und/oder gegebenenfalls Hilfsstoffe wie Konservierungsmittel, Stabilisatoren, Netzmittel, Penetrationsmittel, Emulgatoren, Spreitmittel, Lösungsvermittler, Salze zur Regelung des osmotischen Drucks oder zur Pufferung und/oder Viskositätsregulatoren enthalten.

Derartige Zusätze sind zum Beispiel Tartrat- und Citrat-Puffer, Ethanol, Komplexbildner (wie Äthylendiamin-tetraessigsäure und deren nicht-toxische Salze). Zur Regelung der Viskosität kommen hochmolekulare Polymere in Frage wie beispielweise flüssiges Polyethylenoxid, Carboxymethylcellulosen, Polyvinylpyrrolidone, Dextrane oder Gelatine. Feste Trägerstoffe sind zum Beispiel Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykol).

Ölige Suspensionen für parenterale oder topische (in diesem Falle intraureterale) Anwendungen können vegetabile synthetische oder semisynthetische Öle wie beispielsweise flüssige Fettsäureester mit jeweils 8 bis 22 C-Atomen in den Fettsäureketten, zum Beispiel Palmitin-, Laurin-, Tridecyl-, Margarin-, Stearin-, Arachin-, Myristin-, Behen-, Pentadecyl-, Linol-, Elaidin-, Brasidin-, Eruca- oder Ölsäure, die mit ein- bis dreiwertigen Alkoholen mit 1 bis 6 C-Atomen wie beispielsweise Methanol, Ethanol, Propanol, Butanol, Pentanol oder deren Isomere, Glycol oder Glycerol verestert sein. Derartige Fettsäureester sind beispielsweise handelsübliche ??iglyole, Isopropylmyristat, Isopropylpalmitat, Isopropylstearat, PEG 6-Caprinsäure, Capryl/Caprinsäureester von gesättigten Fettalkoholen, Polyoxyethylenglyceroltrioleate, Ethyloleat, wachsartige Fettsäureester wie künstliches Entenbürzeldrüsenfetz, Kokosfettsäure-isopropylester, Ölsäureoleylester, Ölsäuredecylester, Milchsäureethylester, Dibuthylphthalat, Adipinsäurediisopropylester, Polyol-Fettsäureester u. a. Ebenso geeignet sind Silikonöle verschiedener Viskosität oder Fettalkohole wie Isotridexylalkohol, 2-Octyldodecanol, Cetylstearyl-Alkohol oder Oleylalkohol, Fettsäuren wie beispielsweise Ölsäure. Weiterhin können vegetabile Öle wie Rizinusöl, Mandelöl, Olivenöl, Sesamöl, Baumwollsaatöl, Erdnußöl oder Sojabohnenöl Verwendung finden. Die genannten Stoffe haben zudem die Eigenschaften eines Spreitmittels, das heißt es erfolgt eine besonders gute Verteilung auf der Haut.

Als Lösungsmittel, Gelbildner und Lösungsvermittler kommen in Frage Wasser oder mit Wasser mischbare Lösungsmittel. Geeignet sind zum Beispiel Alkohole wie beispielsweise Ethanol oder Isopropylalkohol, Benzylalkohol, 2-Octyldodecanol, Polyethylenglykole, Phthalate, Adipate, Propylenglyklol, Glycerin, Di- oder Tripropylenglykol, Wachse, ethylcellosolve, Cellosolve, Ester, Morpholine, Dioxan, Dimethylsulfoxid, Dimethylformamid, Tetrahydrofuran, Cyclohexanon etc.

Als Filmbildner können Celluloseether verwendet werden, die sich sowohl in Wasser als auch in organischen Lösungsmitteln lösen bzw. anquellen können und nach dem Trocknen eine Art Film bilden, wie beispielsweise Hydroxypropylcellulose, Methylcellulose, Ethylcellulose oder lösliche Stärken.

Mischformen zwischen Gel- und Filmbildnern sind durchaus ebenfalls möglich. Hier kommen vor allem ionische Makromoleküle zur Anwendung, wie z.B. Natriumcarboxymethylcellulose, Polyacrylsäure, Polymethacrylsäure und deren Salze, Natriumamylopektinsemiglykolat, Alginsäure oder Propylenglykol-Alginat als Natriumsalz, Gummi arabicum, Xanthan-Gummi, Guar-Gummi oder Carrageenan.

Als weitere Formulierungshilfsmittel können eingesetzt werden: Glycerin, Paraffin unterschiedlicher Viskosität, Triethanolamin, Collagen, Allantoin, Novantisolsäure, Parfümöle.

Auch die Verwendung von Tensiden, Emulgatoren oder Netzmitteln kann zur Formulierung notwendig sein, wie zum Beispiel von Na-Laurylsulfat, Fettalkoholethersulfaten, Di-Na-N-lauryl-β-iminodipropionat, polyoxyethyliertes Rizinusöl oder Sorbitan-Monooleat, Sorbitan-Monostearat, Cetylalkohol, Lecithin, Glycerinmonostearat, Polyoxyethylenstearat, Alkylphenolpolyglykolether, Cetyltrimethylammoniumchlorid oder Mono-/Dialkylpolyglykolether-orthophosphorsäuremonoethanolaminsalze.

Stabilisatoren wie Montmorillonite oder kolloidale Kieselsäuren zur Stabilisierung von Emulsionen oder zur Verhinderung des Abbaus der aktiven Substanzen wie Antioxidantien, beispielsweise Tocopherole oder Butylhydroxyanisol, oder Konservierungsmittel, wie p-Hydroxybenzoesäureester, können ebenfalls zur Zubereitung der gewünschten Formulierungen gegebenenfalls erforderlich sein.

Zur Förderung der Penetration enthalten intraureterale Formulierungen vorzugsweise organische, gut verträgliche Lösungsmittel wie Ethanol, Methylpyrrolidon, Polyethylenglykol, Oleylalkohol, Octanol, Linolsäure, Triacetin, Propylenglykol, Glycerin, Solketal oder Dimethylsulfoxid.

Die Herstellung, Abfüllung und die Verschließung der Präparate erfolgt unter den üblichen antimikrobiellen und aseptischen Bedingungen. Auch für topischen beziehungsweise transdermalen Einsatz erfolgt eine Abpackung möglichst in separaten Dosiseinheiten zur Erleichterung der Handhabung, auch hier wie bei parenteralen Formen gegebenenfalls aus Stabilitätsgründen durch separate Abpackung der Wirkstoffe beziehungweise deren Kombinationen als Lyophilisat, gegebenenfalls mit festen Trägerstoffen, und den erforderlichen Lösungsmitteln etc.

### Beispiel 1 - Injektionslösung

50 mg Rolipram werden mit 750 mg NaCl in destilliertem Wasser gelöst, mit 1N HCl auf pH 3,7 eingestellt und mit destilliertem Wasser auf 100 ml aufgefüllt und in 0,5 ml-Ampullen abgepackt.

### Beispiel 2 - Lösung zur topischen Applikation

Aus 500 mg Rolipram, 2 ml Isopropylmyristat und 10 ml Ethanol wird eine Lösung zur topische Applikation bereitet und zu Dosiseinheiten von jeweils 2 ml abgepackt.

Die Wirksamkeit der Arzneimittel für die erfindungsgemäße Lehre wird durch folgende pharmakologischen Untersuchungen belegt:

Frisch bei der Operation entnommener humaner Ureter wird in kleine Streifen geschnitten (ca. 3 x 10 mm). Diese werden dann in ein Bad mit einer Nährlösung installiert, die das Überleben der Organstreifen gewährleistet. Durch ein Ankoppeln der Organstreifen an einen Meßfühler können Längenveränderungen des Organstreifens registriert werden und so Wirkungen von Medikamenten, die in die Organbad-Nährlösung gegeben werden, anhand der Längenänderung (Zu- oder Abnahme) des Organstreifens untersucht werden. Zu Versuchsbeginn werden die Organstreifen mit einem hierzu geeigneten Standardmedikament z. B. Noradrenalin kontrahiert. Nach eingetretener Kontraktion der Organstreifen wird nun in ansteigender Dosierung (10⁻⁷, 10⁻⁶, 10⁻⁵ etc. mol/l) ein Inhibitor einer spezifischen Phosphodiesterase in die Organbadlösung gegeben und die dadurch ausgelöste Relaxation gemessen. Die gewonnen Ergebnisse sind auf den Gesamtorganismus im wesentlichen übertragbar, da humanes Gewebe verwandt wurde und die untersuchten Stoffwechselvorgänge im Gesamtorganismus schneller ablaufen und daher die Medikamente nach schneller wirken.

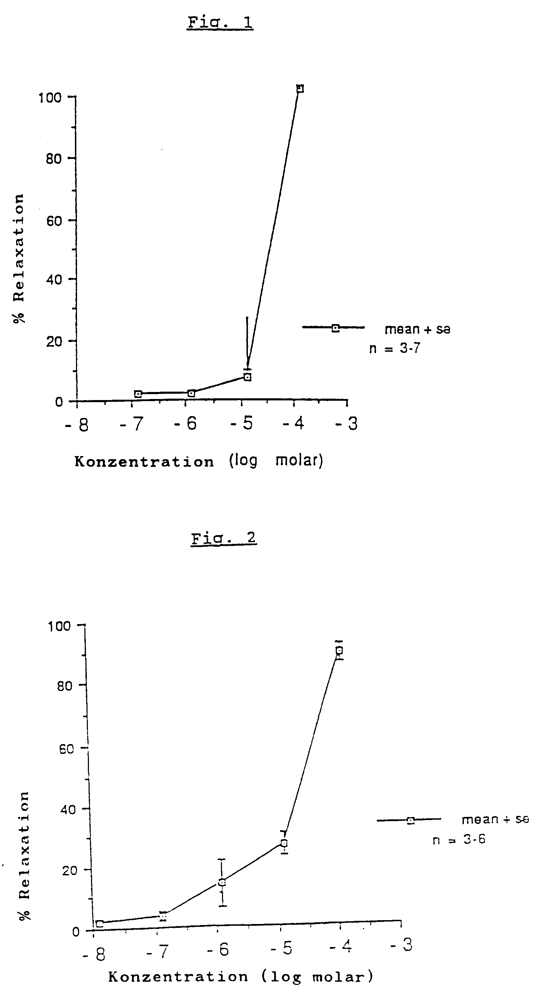

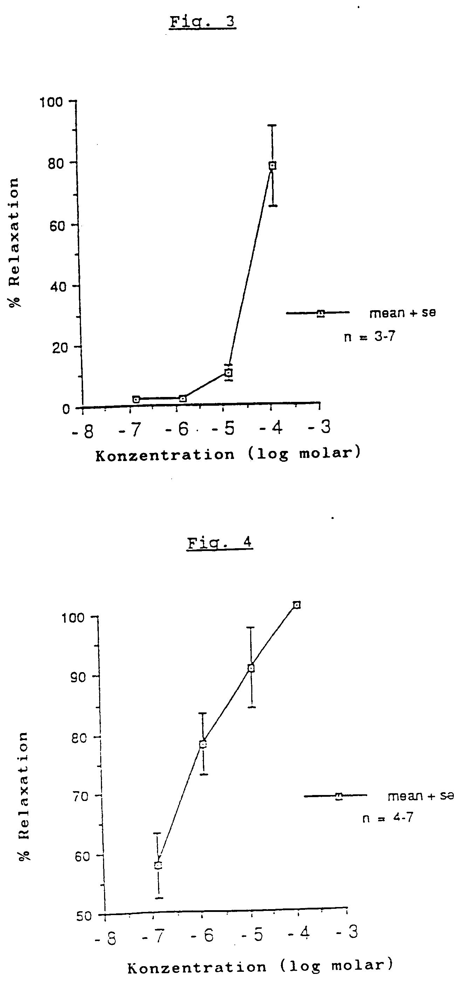

In Fig. 1 bis Fig. 5 sind die Ergebnisse dieser Organbadversuche dargestellt.
Fig. 1 zeigt den relaxierenden Effekt von kumulativ ansteigenden Konzentrationen von Papaverin, einem unspetifischen Phosphodiesterase-Inhibitor, auf humane, mit 80 mM KCl vorkontrahierte Ureter-Streifen. Die Kurve zeigt die Mittelwerte von Messungen an jeweils 3 bis 7 Ureter-Streifen.
Fig. 2 zeigt den relaxierenden Effekt von kumulativ ansteigenden Konzentrationen von Quazinon, einen Inhibitor der sPDE III, auf humane, mit 80 mM KCl vorkontrahierte Ureter-Streifen. Die Kurve zeigt die Mittelwerte von Messungen an jeweils 3 bis 6 Ureter-Streifen.
Fig. 3 zeigt den relaxierenden Effekt von kumulativ ansteigenden Konzentrationen von Zaprinast, einem Inhibitor der sPDE V, auf humane, mit 80 mM KCl vorkontrahierte Ureter-Streifen. Die Kurve zeigt die Mittelwerte von Messungen an jeweils 3 bis 6 Ureter-Streifen.
Fig. 4 zeigt den relaxierenden Effekt von kumulativ ansteigenden Konzentrationen von Rolipram, einem Inhibitor der sPDE IV, auf humane, mit 80 mM KCl vorkontrahierte Ureter-Streifen. Die Kurve zeigt die Mittelwerte von Messungen an jeweils 4 bis 7 Ureter-Streifen.
Fig. 5 zeigt einen Vergleich der relaxierenden Wirkung von Rolipram auf renale und koronare Arterienstreifen.

Die Versuche zu Fig. 5 wurden analog zu den Untersuchungen an Ureterstreifen durchgeführt und belegen in eindeutiger Weise die spezifische relaxierende Wirkung auf das Uretergewebe, während das renale Gefäßsystem überhaupt nicht beeinflußt wird.

Der Nachweis, ob eine Verbindung für den erfindungsgemäßen Zweck geeignet ist, d.h. ein Inhibitor der sPDE IV ist, erfolgt nach bekannten Methoden, wie z.B. beschrieben von Galwan et al., Arch. Pharmacol. 1990, 342, 221-227 oder Nicholson, Br. J Pharmacol., 1989, 79, 889-897, beispielsweise nach folgender allgemeiner Methode:

Frisches, intraoperativ gewonnenes Gewebe wird homogenisiert und dann ultrazentrifugiert. Anschließend wird der Überstand abpipettiert und chromatographiert. Von den 100 Fraktionen von 70 bis 1000 mM (millimolar) werden dann je 5 Ansätze von 30 µl der Enzympräparation hergestellt; jede Enzympräparation einer Fraktion wird mit a) radioaktiv markiertem cAMP, b) radioaktiv markierten cGMP, c) radioaktiv markiertem cAMP plus Calcium plus Calmodulin, d) radioaktiv markiertem cGMP plus Calcium plus Calmodulin, d) radioaktiv markiertem cGMP plus Calcium plus Calmodulin oder e) radioaktiv markiertem cAMP plus cGMP plus Calcium plus Calmodulin versetzt. Nach Inkubation und Beendigung der Reaktion sowie erneuter Zentrifugation wird die Radioaktivität der Proben gemessen. Die Bestimmung der Radioaktivität erlaubt die Berechnung der Enzymaktivität in pmol/ml x min. Die Auftragung der Aktivitätskurve erlaubt die Identifikation von Fraktionen, bei denen die Phophodiesteraseaktivität besonders hoch ist. Die Phospodiesteraseaktivität eines jeden Peaks zeigt eine unterschiedliche Zusammensetzung bezüglich der Aktivität der 5 verschiedenen Ansätze. Diese spezielle Zusammensetzung der Phospodiesteraseaktivität läßt eine Zuordnung zu einer spezifischen Phosphodiesterase (sPDE) zu. Ein Inhibitor einer sPDE ist nun diejenige Substanz, deren Konzentration, die nötig ist, um 50% der Substrathydrolyse zu hemmen (IC₅₀), bei der betreffenden Peakfraktion, die die spezifische Phosphodiesterase enthält, um mindestens 20 mal kleiner ist als bei anderen Peakfraktionen. Dazu werden wiederum, wie oben beschrieben, Enzympräparationen hergestellt. Vor der Inkubation der Enzymansätze nach a) bis e) der Peakfraktionen wird aber nun die zu testende Verbindung zugesetzt. Die erneute Bestimmung und Auftragung der Enzymaktivität erlaubt dann gemäp der oben aufgeführten Definition die Identifikation einer Substanz als Inhibitor der spezifischen Phosphadiesterase.

## Patentansprüche

1. Verwendung von Inhibitoren der Phosphodiesterase IV oder deren Salze zur Herstellung von Arzneimitteln zur Modulation der Motilität und Peristaltik der Hohlorgane des Urogenital- und Gastrointestinaltraktes.

2. Verwendung nach Anspruch 1 zur Behandlung von Nieren-, Harn- oder Gallensteinen.

3. Verwendung nach Anspruch 2 zur Behandlung von Nieren-, Harnleiter- oder Gallenkoliken.

4. Verwendung von razemischem oder optisch aktivem Rolipram nach Anspruch 1 - 3.

5. Verwendung von Inhibitoren der Phosphodiesterase IV oder deren Salzen zur Herstellung von Hilfsstoffen zur Verbesserung der Diagnose mit Kontrastmitteln.

## Claims

1. Use of phosphodiesterase IV inhibitors or salts thereof for the preparation of medicaments for modulating the motility and peristalsis of the hollow organs of the urogenital and gastrointestinal tracts.

2. The use according to claim 1 for the treatment of renal, urinary or biliary calculi.

3. The use according to claim 2 for the treatment of renal, ureteral or biliary colics.

4. Use of racemic or optically active rolipram according to claims 1 to 3.

5. Use of phosphodiesterase IV inhibitors or salts thereof for the preparation of auxiliary agents for improving diagnostics with contrast media.

## Revendications

1. Utilisation d'inhibiteurs de la phosphodiestérase IV ou de ses sels pour la préparation de médicaments pour la modulation de la motilité et du péristaltisme des organes creux de l'appareil urogénital et de l'appareil gastrointestinal.

2. Utilisation selon la revendication 1 pour le traitement des calculs rénaux, urinaires ou biliaires.

3. Utilisation selon la revendication 2 pour le traitement des coliques néphrétiques, urétérales ou hépatiques.

4. Utilisation de Rolipram racémique ou optiquement actif selon les revendications 1 à 3.

5. Utilisation d'inhibiteurs de la phosphodiestérase IV ou de ses sels pour la préparation d'adjuvants pour améliorer le diagnostic avec des substances de contraste.
